(19) Europäisches Patentamt
European Patent Office
Office européen des brevets

(11) Veröffentlichungsnummer: **0 167 730**
**A1**

# (12) EUROPÄISCHE PATENTANMELDUNG

(21) Anmeldenummer: **85104950.2**

(22) Anmeldetag: **24.04.85**

(51) Int. Cl.⁴: **A 01 N 43/70**
C 07 D 251/52

(30) Priorität: **01.06.84 DE 3420488**

(43) Veröffentlichungstag der Anmeldung:
**15.01.86 Patentblatt 86/3**

(84) Benannte Vertragsstaaten:
**AT BE CH DE FR GB IT LI LU NL SE**

(71) Anmelder: **HÜLS AKTIENGESELLSCHAFT**
**- RSP Patente / PB 15 - Postfach 13 20**
**D-4370 Marl 1(DE)**

(72) Erfinder: **Schnurbusch, Horst, Dr.**
**Overwegstrasse 36**
**D-4690 Herne 1(DE)**

(72) Erfinder: **Baltruschat, Helmut, Dr.**
**Am Hagenbach 3**
**D-44o5 Nottuln(DE)**

(54) **Verwendung von Alkoxy-cycloakkylamino-s-triazinen zur selektiven Behandlung von Nutzpflanzen gegen Unkräuter und Schadgräser.**

(57) Die Erfindung betrifft die Verwendung von Alkoxy-cycloalkylamino-s-triazinen der allgemeinen Formel

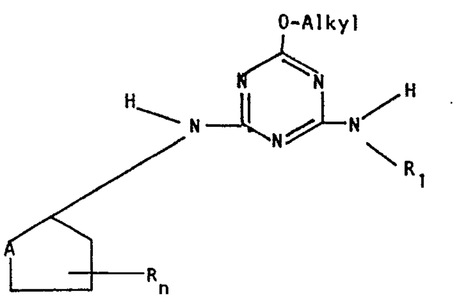

in der

$R_1$ einen gerad- oder verzweigtkettigen Alkyl-, Alkoxy-, Alkoxyalkyl- oder Alkylol-Rest oder ein H-Atom,
R einen Rest aus der Gruppe der Alkyle mit ein bis drei C-Atomen und
A $= -CH_2-$ oder $-CH_2-CH_2-$ bedeuten und
n für A $= -CH_2-$ jeweils 0 oder 1,2,3,4,5 oder 6, bzw. für A $= -CH_2-CH_2-$ jeweils 3,4,5 oder 6

sein können und wobei die Substituenten R an den Cycloalkylringen gleich oder verschieden sein können, in geeigneter Applikationsform zur selektiven Behandlung von Nutzpflanzenkulturen mit breitem Wirkungsspektrum gegen Unkräuter und Schadgräser vor und/oder nach dem Auflaufen der Saat.

EP 0 167 730 A1

Croydon Printing Company Ltd.

Verwendung von Alkoxy-cycloalkylamino-s-triazinen zur selektiven Behandlung von Nutzpflanzen gegen Unkräuter und Schadgräser

Die Verwendung von symmetrischen Triazinen als Herbizid ist bekannt. Ursprünglich als Totalherbizid eingesetzt, liegt heute die Bedeutung dieser Substanzklasse mehr auf dem Gebiet der selektiven Anwendung. Die bekanntesten symmetrischen Triazine sind solche mit einem Cl-Atom, mit einem O-Alkylrest oder mit einem S-Alkylrest. Sie sind ausführlich in der Monographie von R. Wegler, "Chemie der Pflanzenschutz- und Schädlingsbekämpfungsmittel", Bd. 5, Springer-Verlag (1977), S. 336 ff, beschrieben. Bekannt sind z. B. auch die s-Triazine gemäß den DE-OSS 32 02 086, 32 22 147 und 33 22 720.

Des weiteren sind entsprechend der JP-OS 47 23 436 auch unsubstituierte 2-Cyclohexylamino-Derivate von s-Triazinen bekannt und gemäß DE-OS 32 22 147 auch 6-Methylthio-s-triazine, die mit alkylierten, cycloaliphatischen Aminen substituiert sind.

Bei diesen bekannten Wirkstoffen ist die Selektivität gegenüber Nutzpflanzen und die Herbizidwirkung gegen Schadpflanzen nicht voll befriedigend.

Überraschend wurde gefunden, daß bei den Alkoxy-s-triazinen die Verbindungen, die sowohl mit einem gerad- oder verzweigtkettigen, relativ kurzen Amin als auch mit einem reinen oder gegebenenfalls mono-, di-, tri- oder tetraalkylierten cycloaliphatischen Amin substituiert sind, bei gleichwertiger und zum Teil erweiterter Möglichkeit zum Einsatz als selektives Unkrautbekämpfungsmittel eine bessere Schadgräser-Wirkung zeigen als die bisher bekannten Verbindungen. Bemerkenswert ist, daß bei gleichzeitig sehr guter herbizider Wirkung gegen Unkräuter und Schadgräser eine sehr gute Selektivität

in zahlreichen landwirtschaftlichen Kulturen wie Reis (Oryza sativa), Gerste (Hordeum vulgare), Weizen (Triticum aestivum), Roggen (Secale secale), Soja (Glycine max) oder Baumwolle (Gossypium hirsutum) besteht.

Gegenstand der Erfindung ist also die Verwendung von Alkoxycycloalkylamin-s-triazinen der allgemeinen Formel

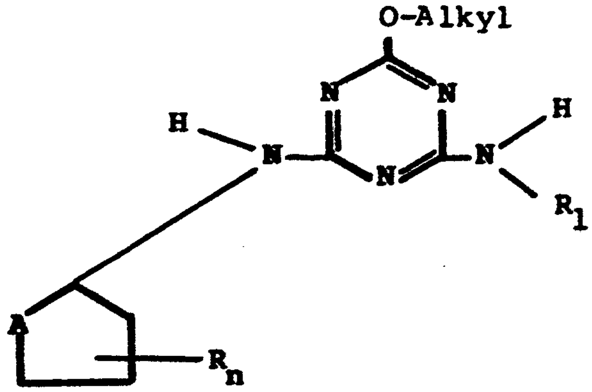

in der

$R_1$ einen gerad- oder verzweigtkettigen Alkyl-, Alkoxy-, Alkoxyalkyl- oder Alkylol-Rest oder ein H-Atom,

R einen Rest aus der Gruppe der Alkyle mit ein bis drei C-Atomen und

A $= -CH_2-$ oder $-CH_2-CH_2-$ bedeuten und

n für A $= -CH_2-$ jeweils 0 oder 1,2,3,4,5 oder 6, bzw. für A $= -CH_2-CH_2-$ jeweils 3,4,5 oder 6

sein können und wobei die Substituenten R an den Cycloalkylringen gleich oder verschieden sein können, in geeigneter Applikationsform zur selektiven Behandlung von Nutzpflanzenkulturen mit

breitem Wirkungsspektrum gegen Unkräuter und Schadgräser vor und/oder nach dem Auflaufen der Saat.
Vorzugsweise erfolgt die Applikation vor Auflaufen der Saat.

Die Ketten, die von $R_1$ gebildet werden, sollten - bei Nichtberücksichtigung der H-Atome und unabhängig vom Verzweigungsgrad - nicht mehr als 15 C- (bzw. O) Atome aufweisen.

Besonders bemerkenswert bei dieser Stoffgruppe ist die sehr gute Wirkung gegen Hirsearten, wie Echinochloa crus-galli, Echinochloa colonum, Digitaria sanguinalis, Setaria viridis und Setaria faberi.
Bekanntlich sind bestimmte Hirsearten wie Echinochloa spec. wegen ihrer botanischen Verwandtschaft zu Reis in dieser Kultur besonders schwierig zu bekämpfen. In der JP-OS 47 23 436 sind zwar kontitutionell ähnliche Stoffe mit herbizider Wirkung gegen Echinochloa spec. bei gleichzeitiger Reisselektivität beschrieben worden; sie werden jedoch bezüglich ihrer herbiziden Wirksamkeit deutlich von den erfindungsgemäßen Verbindungen übertroffen. So läßt sich z. B. das erfindungsgemäße 2-Methoxy-4-cyclopentylamino-6-ethylamino-s-triazin besser zur selektiven Unkrautbekämpfung in Reis einsetzen als das 2-Methoxy-4-cyclohexylamino-6-ethylamino-s-triazin, das in der JP-OS 47 23 436 beschrieben wird. Darüber hinaus besitzen die erfindungsgemäß angewendeten Verbindungen überraschenderweise bei der Unkrautbekämpfung in Weizen, Gerste und Roggen eine hohe Selektivität bei gleichzeitig besserer Wirkung gegen Schadgräser, insbesondere Ackerfuchsschwanz und Windhalm, als die vorbenannten Verbindungen aus der JP-OS 47 23 436. Sinngemäß das gleiche gilt für die Selektivität und herbizide Wirksamkeit der erfindungsgemäßen Verbindungen in Baumwolle, Mais, Sojabohnen und Erdnüssen. s-Triazine mit guter Wirkung gegen alle wichtigen Hirsearten sowie Ackerfuchsschwanz, Windhalm u. a. Schadgräser bei gleichzeitiger Selektivität in

Getreidekulturen und besonders in Reis sind bisher nicht bekannt.
Die erfindungsgemäß angewendeten Verbindungen stellen somit eine wertvolle Bereicherung der herbiziden Mittel zur selektiven Unkrautbekämpfung dar.

Gegenüber anderen in der Literatur bekanntgewordenen Reisherbiziden wie Propachlor (N-Isopropyl-$\alpha$-chloracetanilid) weisen die erfindungsgemäß angewendeten Verbindungen den Vorteil der für s-Triazine bekannten Breitenwirkung sowohl gegen dikotyle als auch monokotyle Unkräuter auf.

Der erfindungsgemäße Einsatz der Verbindungen ist nicht nur auf Kulturen der o. g. Pflanzenarten beschränkt, er kann auch erfolgreich in Kulturen anderer Pflanzen erfolgen.

In Abhängigkeit von der Konzentration können die Verbindungen auch zur Totalunkrautbekämpfung, z. B. auf Industrie- und Gleisanlagen oder auf Wegen und Plätzen mit und ohne Baumbewuchs, eingesetzt werden. Auch zur Unkrautbekämpfung in Dauerkulturen, wie z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen sind die erfindungsgemäßen Verbindungen geeignet.

Bei einigen Pflanzenarten wirken einige der erfindungsgemäßen Verbindungen hemmend auf das Längenwachstum, ohne jedoch zu Ertragsminderungen zu führen; sie können deshalb auch zusätzlich als wachstumsregulierende Mittel eingesetzt werden. Manche der neuen Verbindungen können auch als Defoliants, Desiccants, Krautabtötungsmittel und Keimhemmungsmittel verwendet werden.

Die Herstellung der erfindungsgemäß verwendeten Triazine kann nach an sich bekannten Methoden erfolgen, indem man Cyanurchlorid - vorzugsweise in einem Lösungsmittel oder Lösungsmittelgemisch, z. B. in Aceton, Aceton/Wasser, Dioxan, Dioxan/Wasser, Tetrahydrofuran oder in Dimethylformamid - mit den entsprechenden Aminen bei der jeweils zweckmäßigen Temperatur, die gewöhnlich zwischen -30 °C und +50 °C liegt, umsetzt.

Zur selektiven Substitution am Triazingerüst arbeitet man nach der für die stufenweise Substitution in 1,3,5-Triazinen bekannten Arbeitsweise.

Die zuerst erhaltenen Chlor-Derivate werden in dem entsprechenden Alkohol oder anderen Lösungsmitteln mit Natriumalkylat zum entsprechenden Alkoxyderivat umgesetzt.

Zu der nicht beanspruchten Herstellung wird daher nun ein Beispiel angeführt.

Beispiel 1

Darstellung des 2-Methoxy-4-cyclopentylamino-6-ethylamino-s-triazin

Ansatz:

| | | | |
|---|---|---|---|
| 180 g | 2-Methoxy-4.6-dichlor-s-triazin | 1,00 Mol |
| 85 g | Cyclopentylamin | 1,00 Mol |
| 184,5 g | 50 %ige Ethylaminlösung | 2,05 Mol |
| 133,5 g | 30 %ige NaOH | 1,00 Mol |
| 500 ml | Dioxan | |

In die klare Lösung von 2-Methoxy-4.6-dichlor-s-triazin in Dioxan wird bei 20 °C unter Rühren das Cyclopentylamin eingetropft. Eintropfdauer: 30 min; der Ansatz muß gekühlt werden. Anschließend wird in 60 min die berechnete Menge

30 %iger NaOH unter Rühren eingetropft. Dabei soll der pH-Wert von 8,0 nicht überschritten werden. Nach Zugabe der Ethylamin-Lösung bei Raumtemperatur wird unter Rühren auf 70 °C erwärmt. Beim Aufheizen ist eine Reaktionswärme zu beobachten. Zur Beendigung der Reaktion wird 30 min bei 70 °C gerührt. Das Dioxan wird danach abdestilliert und der Rückstand mit heißem Wasser chloridfrei gewaschen. Es werden 203 g Produkt mit dem Schmelzpunkt von 95 °C erhalten, das ist eine Ausbeute von 85 %, bezogen auf eingesetztes Methoxy-dichlortriazin.

Tabelle 1a: Liste der hergestellten Methoxycycloalkyl-s-triazine

Beispiel 1 = 2-Amino-4-cyclopentylamino-6-methoxy-s-triazin

Beispiel 2 = 2-Cyclopentylamino-4-methoxy-6-methylamino-s-triazin

Beispiel 3 = 2-Cyclopentylamino-4-ethylamino-6-methoxy-s-triazin

Beispiel 4 = 2-Cyclopentylamino-4-isopropylamino-6-methoxy-s-triazin

Beispiel 5 = 2-Cyclopentylamino-4-methoxy-6-propylamino-s-triazin

Beispiel 8 = 2-Cyclopentylamino-4-methoxy-6-2'-methoxy-ethylamino-s-triazin

Beispiel 9 = 2-Cyclopentylamino-4-methoxy-6-3'-methoxy-propylamino-s-triazin

Beispiel 10 = 2-Cyclopentylamino-4-3'-ethoxy-propylamino-6-methoxy-s-triazin

Beispiel 12 = 2-Cyclopentylamino-4-3'n-hexoxy-propylamino-6-methoxy-s-triazin

Beispiel 13 = 2-Cyclopentylamino-4-3'-isopropoxy-propyl-amino-6-methoxy-s-triazin

Beispiel 14 = 2-Cyclopentylamino-4-3'-isobutoxy-propylamino-6-methoxy-s-triazin

Beispiel 15 = 2-Amino-4-methoxy-6-TMCP-amino-s-triazin

Beispiel 17 = 2-Ethylamino-4-methoxy-6-TMCP-amino-s-triazin

Beispiel 18 = 2-Isopropylamino-4-methoxy-6-TMCP-amino-s-triazin

Beispiel 20 = 2-tert.-Butylamino-4-methoxy-6-TMCP-amino-s-triazin

Beispiel 21 = 2-Ethanolamino-4-methoxy-6-TMCP-amino-s-triazin

---

TMCP ≙ 2.2.4-(2.4.4)-Trimethylcyclopentyl

TMC ≙ 3.3.5-Trimethylcyclohexyl (cis, trans-Gemisch)

Beispiel 22 = 2-Methoxy-4-2'-methoxy-ethylamino-6-TMCP-amino-s-triazin

Beispiel 23 = 2-Methoxy-4-3'-methoxy-propylamino-6-TMCP-amino-s-triazin

Beispiel 24 = 2-3'-Ethoxy-propylamino-4-methoxy-6-TMCP-amino-s-triazin

Beispiel 25 = 2-3'-n-Butoxy-propylamino-4-methoxy-6-TMCP-amino-s-triazin

Beispiel 26 = 2-3'-n-Hexoxy-propylamino-4-methoxy-6-TMCP-amino-s-triazin

Beispiel 27 = 2-3'-Isopropoxy-propylamino-4-methoxy-6-TMCP-amino-s-triazin

Beispiel 28 = 2-Isobutoxy-propylamino-4-methoxy-6-TMCP-amino-s-triazin

Beispiel 29 = 2-Amino-4-methoxy-6-TMC-amino-s-triazin

Beispiel 30 = 2-Methylamino-4-methoxy-6-TMC-amino-s-triazin

Beispiel 31 = 2-Ethylamino-4-methoxy-6-TMC-amino-s-triazin

Beispiel 32 = 2-Isopropylamino-4-methoxy-6-TMC-amino-s-triazin

Beispiel 36 = 2-Methoxy-4-2'-methoxy-ethylamino-6-TMC-amino-s-triazin

Beispiel 38 = 2-3'-Ethoxy-propylamino-4-methoxy-6-TMC-amino-s-triazin

Beispiel 46 = 2-Ethanolamino-4-methoxy-6-cis-TMC-amino s-triazin

Beispiel 50 = 2-Ethylamino-4-methoxy-6-cis-TMC-amino-s-triazin

Beispiel 51 = 2-Methoxy-4-2'-methoxy-ethylamino-6-TMC-amino-s-triazin

Beispiel 53 = 2-3'-Ethoxy-propylamino-4-methoxy-6-trans-TMC-amino-s-triazin

Beispiel 58 = 2-Amino-4-4'-tert.-butyl-cyclohexylamino-6-methoxy-s-triazin

---

TMCP ≙ 2.2.4-(2.4.4)-Trimethylcyclopentyl

TMC ≙ 3.3.5-Trimethylcyclohexyl (cis, trans-Gemisch)

Beispiel 60 = 2-4'-tert.-Butyl-cyclohexylamino-4-ethylamino-6-methoxy-s-triazin

Beispiel 61 = 2-4'-tert.-Butyl-cyclohexylamino-4-isopropylamino-6-methoxy-s-triazin

Beispiel 65 = 2-4'-tert.-Butyl-cyclohexylamino-4-2'-methoxy-ethylamino-6-methoxy-s-triazin

Beispiel 73 = 2-Cyclopentylamino-4-ethoxy-6-methylamino-s-triazin

Beispiel 74 = 2-Cyclopentylamino-4-ethoxy-6-ethylamino-s-triazin

Beispiel 75 = 2-Cyclopentylamino-4-ethoxy-6-isopropylamino-s-triazin

Beispiel 76 = 2-Cyclopentylamino-4-ethoxy-6-propylamino-s-triazin

Beispiel 78 = 2-Cyclopentylamino-4-ethanolamino-6-ethoxy-s-triazin

Beispiel 79 = 2-Cyclopentylamino-4-ethoxy-6-2'-methoxy-ethylamino-s-triazin

Beispiel 80 = 2-Cyclopentylamino-4-ethoxy-6-3'-methoxy-propylamino-s-triazin

Beispiel 81 = 2-Cyclopentylamino-4-ethoxy-6-3'-ethoxy-propylamino-s-triazin

Beispiel 82 = 2-Cyclopentylamino-4-ethoxy-6-3'-propoxy-propylamino-s-triazin

Beispiel 84 = 2-Cyclopentylamino-4-ethoxy-6-3'-isopropoxy-propylamino-s-triazin

Beispiel 86 = 2-Amino-4-ethoxy-6-TMCP-amino-s-triazin

Beispiel 89 = 2-Ethoxy-4-ethylamino-6-TMCP-amino-s-triazin

Beispiel 90 = 2-Ethoxy-4-isopropylamino-6-TMCP-amino-s-triazin

Beispiel 91 = 2-Ethoxy-4-propylamino-6-TMCP-amino-s-triazin

Beispiel 92 = 2-tert.-Butylamino-4-ethoxy-6-TMCP-amino-s-triazin

Beispiel 94 = 2-Ethoxy-4-2'-methoxy-ethylamino-6-TMCP-amino-s-triazin

---

TMCP ≙ 2.2.4-(2.4.4)-Trimethylcyclopentyl

TMC ≙ 3.3.5-Trimethylcyclohexyl (cis, trans-Gemisch)

Beispiel 96 = 2-Ethoxy-4-3'-ethoxy-propylamino-6-TMCP-amino-s-triazin

Beispiel 104 = 2-Ethoxy-4-ethylamino-6-TMC-amino-s-triazin

Beispiel 109 = 2-Ethoxy-4-2'-methoxy-ethylamino-6-TMC-amino-s-triazin

Beispiel 111 = 2-Ethoxy-4-3'-ethoxy-propylamino-6-TMC-amino-s-triazin

Beispiel 120 = 2-Ethoxy-4-isopropylamino-6-TMC-amino-s-triazin

Beispiel 124 = 2-Ethoxy-4-2'-methoxy-ethylamino-6-TMC-amino-s-triazin

Beispiel 126 = 2-Ethoxy-4-3'-ethoxy-propylamino-6-TMC-amino-s-triazin

Beispiel 132 = 2-4'-tert.-Butyl-cyclohexylamino-4-ethoxy-6-methylamino-s-triazin

Beispiel 134 = 2-4'-tert.-Butyl-cyclohexylamino-4-ethoxy-6-ethylamino-s-triazin

Beispiel 135 = 2-4'-tert.-Butyl-cyclohexylamino-4-ethoxy-6-isopropylamino-s-triazin

Beispiel 136 = 2-4'-tert.-Butyl-cyclohexylamino-4-ethoxy-6-propylamino-s-triazin

---

TMCP ≙ 2.2.4-(2.4.4)-Trimethylcyclopentyl

TMC ≙ 3.3.5-Trimethylcyclohexyl (cis, trans-Gemisch)

Tabelle 1b: Charakterisierung der hergestellten Verbindungen durch den Schmelzpunkt

| Beispiel | Schmelzpunkt (°C) | Beispiel | Schmelzpunkt (°C) |
|---|---|---|---|
| 1 | 160 - 170 | 53 | 40 (harzartig) |
| 2 | 95 - 105 | 58 | 158 - 165 |
| 3 | 90 - 95 | 60 | 135 - 140 |
| 4 | 102 - 111 | 61 | 125 - 136 |
| 5 | 35 (harzartig) | 65 | 45 (harzartig) |
| 8 | 40 (harzartig) | 73 | 65 (harzartig) |
| 9 | 81 - 106 | 74 | 74 - 81 |
| 10 | 73 - 97 | 75 | 35 (harzartig) |
| 12 | 40 (harzartig) | 76 | 60 (harzartig) |
| 13 | 50 (harzartig) | 78 | 91 - 107 |
| 14 | 35 (harzartig) | 79 | 40 (harzartig) |
| 15 | 125 - 136 | 80 | 45 (harzartig) |
| 17 | 38 (harzartig) | 81 | 45 (harzartig) |
| 18 | 40 (harzartig) | 82 | 80 - 85 |
| 20 | 45 - 50 | 84 | 35 (harzartig) |
| 21 | 40 (harzartig) | 86 | 40 (harzartig) |
| 22 | 40 (harzartig) | 89 | 30 (harzartig) |
| 23 | 32 (harzartig) | 90 | 38 (harzartig) |
| 24 | 38 (harzartig) | 91 | 45 (harzartig) |
| 25 | 35 (harzartig) | 92 | 35 (harzartig) |
| 26 | 40 (harzartig) | 94 | 40 (harzartig) |
| 27 | 38 (harzartig) | 96 | 45 (harzartig) |
| 28 | 45 (harzartig) | 104 | 104 - 110 |
| 29 | 120 - 131 | 109 | 35 (harzartig) |
| 30 | 55 - 61 | 111 | 30 (harzartig) |
| 31 | 65 - 75 | 120 | 30 (harzartig) |
| 32 | 50 (harzartig) | 124 | 30 (harzartig) |
| 36 | 105 - 110 | 126 | 35 (harzartig) |
| 38 | 105 - 111 | 132 | 130 - 147 |
| 46 | 38 (harzartig) | 134 | 110 - 140 |
| 50 | 45 (harzartig) | 135 | 105 - 125 |
| 51 | 35 (harzartig) | 136 | 125 - 145 |

Applikation der Wirkstoffe

Die Wirkstoffe der erfindungsgemäß eingesetzten Verbindungen können je nach Kultur in einer Menge von 0,125 bis 10 kg/ha, vorzugsweise von 0,5 bis 2,5 kg/ha, appliziert werden. Es können sowohl Racemate entsprechend einer Formel (Verbindung mit einem Wert für $R_1$, $R_2$ und R) als auch Gemische von erfindungsgemäßen Verbindungen (d. h. solche mit verschiedenen Bedeutungen für $R_1$ und/oder $R_2$ und/oder R) zur Bekämpfung von Unkräutern und Ungräsern eingesetzt werden.

Außerdem ist es üblich, bei der Applikation ein oder mehrere Hilfsmittel aus der Gruppe der Träger-, Verdünnungs-, Lösungs-, Netz-, Haft-, Dispergier- und Emulgiermittel den Wirkstoffen zuzusetzen.

Auch die Zumischung anderer Herbizide bzw. der gleichzeitige Einsatz von Fungiziden, Insektiziden, Wachstumsregulatoren usw. mit den erfindungsgemäßen Wirkstoffen bzw. Wirkstoffgemischen ist möglich.

Schließlich können die erfindungsgemäßen Wirkstoffe oder Wirkstoffgemische, ggf. mit einem oder mehreren Vertretern aus den o. g. Stoffgruppen, Mineraldüngern oder Bodenverbesserungsmitteln zugesetzt und mit ihnen zusammen verteilt werden.

Im Vorauflauf erfolgt die Applikation als Vorsaatapplikation mit Einarbeitung des Wirkstoffes in den Boden oder auf die Bodenoberfläche vor dem Keimen der Samen oder im Nachauflauf auf die Blattoberfläche, vorzugsweise im zweiten bis dritten Blattstadium.

Als Vergleichssubstanzen für die Gewächshaus-Versuche dienten:

A = 2-Methoxy-4-cyclohexylamino-6-ethylamino-s-triazin
B = 2-Ethoxy-4-cyclohexylamino-6-ethylamino-s-triazin
C = 2-Chlor-4-ethylamino-6-isopropylamino-s-triazin
D = 2-Chlor-4-cyclopentylamino-6-ethylamino-s-triazin
E = 2-Methylthio-4-cyclopentylamino-6-ethylamino-s-triazin

Gewächshaus-Versuchsreihe mit 4, 3 und 2 kg/ha

Für die meisten der erfindungsgemäß eingesetzten Verbindungen wurden Gewächshaus-Versuche mit einer Aufwandmenge von 4, 3 und 2 kg/ha, bezogen auf reinen Wirkstoff, an verschiedenen Testpflanzen durchgeführt.

Die jeweils benötigten Wirkstoffmengen wurden dabei in 1 000 l Wasser/ha suspendiert. Jeweils 3 Wochen nach der Behandlung der Kulturen mit dem Wirkstoff wurde bonitiert. Die Auswertung der Versuchsergebnisse erfolgte durch die übliche Benotung, bei der

1 = volle Wirkung = Abtötung der Pflanzen und
5 = keine Wirkung = Pflanzen wie unbehandelt

bedeuten.

Die in den Versuchen getesteten Pflanzenarten sind in Tabelle 1 aufgeführt.

Tabelle 1: Botanische Namen der Testpflanzen

| lateinische Bezeichnung | Code | deutsche Bezeichnung |
|---|---|---|
| Alopecurus myosuroides | ALOMY | Ackerfuchsschwanz |
| Amaranthus retroflexus | AMARE | Amaranth (zurückgekrümmte) |
| Apera spica-venti | APESV | Windhalm |
| Avena fatua | AVEFA | Flughafer |
| Avena sativa | AVESA | Hafer |
| Beta vulgaris | BETVU | Zuckerrübe |
| Brassica napus | BRANA | Winterraps |
| Chenopodium album | CHEAL | Weißer Gänsefuß |
| Digitaria sanguinalis | DIGSA | Blut-Fingerhirse |
| Echinochloa crus-galli | ECHCG | Hühnerhirse |
| Glycine max | GLYMA | Sojabohnen |
| Gossypium herbaceum | GOSHE | Baumwolle |
| Helianthus annuus | HELAN | Sonnenblume |
| Hordeum vulgare | HORVU | Gerste |
| Lamium purpureum | LAMPU | Taubnessel |
| Lolium perenne | LOLPE | Deutsches Weidelgras |
| Lycopersicum esculentum | LYCES | Tomate |
| Matricaria spp. | MATSS | Kamille |
| Oryza sativa | ORYSA | Reis |
| Secale secale | SECSE | Roggen |
| Setaria faberi | SETFA | Faber's Borstenhirse |
| Setaria viridis | SETVI | Grüne Borstenhirse |
| Sinapis arvensis | SINAR | Ackersenf |
| Sorghum halapense | SORHA | Johnsongras |
| Stellaria media | STEME | Vogelmiere |
| Triticum aestivum | TRIAE | Weizen |
| Zea mays | ZEAMA | Mais |

Die erfindungsgemäß eingesetzten Verbindungen zeigen sowohl im Vor- als auch im Nachauflauf (Tabelle 2) eine hervorragende Wirkung gegen Schadgräser, wie z. B. Alopecurus myosuroides, Avena fatua und Echinochloa crus-galli und zweikeimblättrige Unkräuter, wie z. B. Sinapis arvensis, Matricaria spp. und Stellaria media. Neben dem breiten Wirkungsspektrum fällt aber die für Triazin-Verbindungen überraschend hohe Verträglichkeit in verschiedenen Kulturpflanzen wie Winterweizen, Soja oder Reis im Vor- und/oder Nachauflauf auf, welche die Vergleichsverbindungen nicht in diesem Maße zeigen.

Gewächshaus-Versuchsreihe mit 1, 0,5, 0,25 und 0,125 kg/ha

Wegen der vorhandenen hohen herbiziden Wirkung der erfindungsgemäß eingesetzten Verbindungen wurden einige zusätzlich in noch niedrigeren Aufwandmengen (1, 0,5, 0,25 und 0,125 kg/ha) im Vor- und Nachauflauf geprüft (Tabelle 3).

Die erfindungsgemäß eingesetzten Verbindungen zeigen ein weites Wirkungsspektrum und übertreffen in wichtigen Indikationen (u. a. Alopecurus myosuroides, Apera spica-venti, Avena fatua, Echinochloa crus-galli, Setaria faberi, Setaria viridis, Digitaria sanguinalis, Amaranthus retroflexus, Chenopodium album, Lamium purpureum, Sinapis arvensis) die Wirkung der konstitutionell ähnlichen Vergleichsverbindungen A und B sowohl im Vor- als auch Nachauflauf deutlich. Gegenüber dem Vergleichsmittel C besteht eine bessere Wirkung der erfindungsgemäßen Verbindungen gegen Hirsearten (Echinochloa crus-galli, Digitaria sanguinalis, Setaria viridis, Setaria sanguinalis), welche in einigen wichtigen Kulturen wie Baumwolle, Soja und Mais als Schadgräser bedeutsam sind.

Gleichzeitig wird von den erfindungsgemäß eingesetzten Verbindungen eine hohe Kulturverträglichkeit in Weizen, Gerste, Roggen, Reis, Mais, Baumwolle und Soja erzielt, während das Vergleichsmittel C bekanntlich nur in Mais, nicht aber in den übrigen aufgeführten Kulturarten hinreichende Kulturselektivität besitzt.

Selektive herbizide Wirkung auf Reis im Nachauflauf-Verfahren

25 Tage alte Reissetzlinge wurden - jeweils doppelt zu einer Pflanze gesetzt - in große quadratische Eternitcontainer verpflanzt. Zwischen die Reihen der Reispflanzen wurden Samen der in Reis bedeutsamen Wildhirse Echinochloa crus-galli ("Watergrass", Sumpfvarietät) gesät. Die Schalen wurden gut bewässert und bei einer Temperatur von ca. 25 °C und 95 % rel. Luftfeuchtigkeit gehalten. Nach 10 Tagen, also im 2-3 Blattstadium der Wildhirse, wurde die Erde in der Schale mit einer 2,5 cm hohen Schicht Wasser bedeckt. Der Wirkstoff wurde in einer geeigneten Formulierung (Emulsions-, Suspensionskonzentrat oder wettable Powder) mittels einer Pipette zwischen die Pflanzenreihen appliziert, wobei der Wirkstoff so mit Wasser verdünnt war, daß die Applikationsmenge 3, 2, 1 und 0,5 kg a. i./ha entsprach. Der Versuch wurde nach 4 Wochen ausgewertet. Für die Bonitierung wurden die Frischgewichte der behandelten Pflanzen mit den Kontrollpflanzen in Beziehung gesetzt und die jeweiligen Wirkungsgrade in % wiedergegeben.

Aus Tabelle 3 ist ersichtlich, daß die erfindungsgemäß eingesetzten Verbindungen den konstitutionell ähnlichen Verbindungen A und B in der Wirkung gegen Echinochloa crus-galli, dem wichtigsten Schadgras in Reis, deutlich überlegen sind. Die Wirkung gegen diese Wildhirse beträgt selbst in der niedrigsten geprüften Wirkstoffmenge bei einigen erfindungsgemäß eingesetzten Verbindungen noch 100 %, während die Vergleichssubstanz A bei dieser Aufwandmenge 83 %, die Vergleichssubstanz B nur 5 % Wirkung erreicht. Auch gegenüber den konstitutionell ähnlichen Vergleichsmitteln D und E bestehen insofern Vorteile, als die erfindungsgemäßen Verbindungen gegenüber dem Vergleichsmittel E eine bessere Wirkung gegen Echinochloa crus-galli aufweisen und die Vergleichsverbindung D in Reis nicht selektiv ist. Die Reis-Selektivität der erfindungsgemäß eingesetzten Verbindungen ist mit der der Vergleichssubstanz A und B voll vergleichbar. Die Vergleichsverbindung C weist dagegen unter den beschriebenen Versuchsbedingungen keine Reis-Selektivität auf.

Tabelle 2:

Selektivie herbizide Wirkung der erfindungsgemäßen Verbindungen in den Aufwandmengen 4, 3 und 2 kg a.i./ha vor dem Auflaufen (VA) bzw. nach dem Auflaufen (NA) der Testpflanzen

| Verbindung Nr. | kg Wirkstoff a.i./ha | SINAP | | LYCES | | ECHCG | | TRIAE | | AVEFA | | ALOMY | | GLYMA | | LOLPE | | STEME | | MATSS | | ORYSA | |
|---|---|---|---|---|---|---|---|---|---|---|---|---|---|---|---|---|---|---|---|---|---|---|---|
| | | VA | NA | VA | NA | VA | NA | VA | NA | VA | NA | VA | NA | VA | NA | VA | NA | VA | NA | VA | NA | VA | NA |
| A (bekannt) | 4 | 1 | 1 | 1 | 1 | 1 | 1 | 1 | 1 | 1 | 1 | 1 | 1 | 1 | 1 | 2 | 1 | 1 | 1 | 1 | 1 | 2 | 2 |
| | 3 | 1 | 1 | 1 | 1 | 1 | 1 | 1 | 1 | 1 | 1 | 1 | 1 | 1 | 1 | 2 | 1 | 1 | 1 | 1 | 1 | 2 | 2 |
| | 2 | 1 | 1 | 1 | 1 | 1 | 1 | 1 | 1 | 1 | 1 | 1 | 1 | 1 | 1 | 5 | 1 | 1 | 1 | 1 | 1 | 5 | 2 |
| B (bekannt) | 4 | 1 | 1 | 1 | 1 | 1 | 1 | 1 | 1 | 1 | 1 | 1 | 1 | 2 | 1 | 3 | 1 | 1 | 1 | 1 | 1 | 1 | 1 |
| | 3 | 1 | 1 | 1 | 1 | 1 | 1 | 1 | 1 | 1 | 1 | 1 | 1 | 2 | 1 | 3 | 1 | 1 | 1 | 1 | 1 | 4 | 1 |
| | 2 | 1 | 1 | 1 | 1 | 1 | 1 | 1 | 1 | 1 | 1 | 1 | 1 | 2 | 1 | 5 | 1 | 1 | 1 | 1 | 1 | 5 | 1 |
| C (bekannt) Atrazin | 4 | 1 | 1 | 1 | 1 | 1 | 1 | 1 | 1 | 1 | 1 | 1 | 1 | 1 | 1 | 1 | 1 | 1 | 1 | 1 | 1 | 1 | 1 |
| | 3 | 1 | 1 | 1 | 1 | 1 | 1 | 1 | 1 | 1 | 1 | 1 | 1 | 1 | 1 | 1 | 1 | 1 | 1 | 1 | 1 | 2 | 1 |
| | 2 | 1 | 1 | 1 | 1 | 1 | 1 | 1 | 1 | 1 | 1 | 1 | 1 | 1 | 1 | 1 | 1 | 1 | 1 | 1 | 1 | 2 | 1 |
| 1 | 4 | 1 | 1 | 1 | 1 | 1 | 1 | 2 | 2 | 1 | 1 | 1 | 1 | 1 | 1 | 1 | 1 | 1 | 1 | 1 | 1 | 5 | 5 |
| | 3 | 1 | 1 | 1 | 1 | 1 | 1 | 2 | 2 | 1 | 1 | 1 | 1 | 1 | 1 | 1 | 1 | 1 | 1 | 1 | 1 | | |
| | 2 | 1 | 1 | 1 | 1 | 1 | 1 | 2 | 2 | 1 | 1 | 1 | 1 | 2 | 1 | 1 | 1 | 1 | 1 | 1 | 1 | | |
| 2 | 4 | 1 | 1 | 1 | 1 | 1 | 1 | 1 | 1 | 1 | 1 | 1 | 1 | 1 | 1 | 1 | 1 | 1 | 1 | 1 | 1 | 3 | 3 |
| | 3 | 1 | 1 | 1 | 1 | 1 | 1 | 1 | 1 | 1 | 1 | 1 | 1 | 1 | 1 | 1 | 1 | 1 | 1 | 1 | 1 | 4 | 3 |
| | 2 | 1 | 1 | 1 | 1 | 1 | 1 | 1 | 1 | 1 | 1 | 1 | 1 | 1 | 1 | 1 | 1 | 1 | 1 | 1 | 1 | 5 | 5 |
| 3 | 4 | 1 | 1 | 1 | 1 | 1 | 1 | 1 | 1 | 1 | 1 | 1 | 1 | 1 | 1 | 1 | 1 | 1 | 1 | 1 | 1 | 3 | 3 |
| | 3 | 1 | 1 | 1 | 1 | 1 | 1 | 1 | 1 | 1 | 1 | 1 | 1 | 1 | 1 | 1 | 1 | 1 | 1 | 1 | 1 | 4 | 3 |
| | 2 | 1 | 1 | 1 | 1 | 1 | 1 | 1 | 1 | 1 | 1 | 1 | 1 | 1 | 1 | 1 | 1 | 1 | 1 | 1 | 1 | 5 | 5 |
| 4 | 4 | 1 | 1 | 1 | 1 | 1 | 1 | 1 | 2 | 1 | 1 | 1 | 1 | 1 | 1 | 2 | 1 | 1 | 1 | 1 | 1 | 5 | 5 |
| | 3 | 1 | 1 | 1 | 1 | 1 | 1 | 1 | 2 | 1 | 1 | 1 | 1 | 1 | 1 | 2 | 1 | 1 | 1 | 1 | 1 | | |
| | 2 | 1 | 1 | 1 | 1 | 1 | 1 | 3 | 2 | 1 | 1 | 1 | 1 | 1 | 1 | 2 | 1 | 1 | 1 | 1 | 1 | | |
| 5 | 4 | 1 | 1 | 1 | 1 | 1 | 1 | 4 | 1 | 1 | 1 | 1 | 1 | 3 | 1 | 4 | 1 | 1 | 1 | 1 | 1 | 5 | 5 |
| | 3 | 1 | 1 | 1 | 1 | 1 | 1 | 5 | 1 | 1 | 1 | 1 | 1 | 3 | 1 | 5 | 1 | 1 | 1 | 1 | 1 | | |
| | 2 | 1 | 1 | 1 | 1 | 1 | 1 | | 1 | 1 | 1 | 1 | 1 | 3 | 1 | | 3 | 1 | 1 | 1 | 1 | | |
| 8 | 4 | 1 | 1 | 1 | 1 | 1 | 1 | 5 | 1 | 1 | 1 | 1 | 1 | 3 | 1 | 2 | 1 | 1 | 1 | 1 | 1 | 5 | 5 |
| | 3 | 1 | 1 | 1 | 1 | 1 | 1 | | 1 | 1 | 1 | 1 | 1 | 3 | 1 | 2 | 1 | 1 | 1 | 1 | 1 | | |
| | 2 | 1 | 1 | 1 | 1 | 1 | 1 | | 2 | 1 | 1 | 1 | 1 | 3 | 1 | 5 | 2 | 1 | 1 | 1 | 1 | | |
| 9 | 4 | 1 | 1 | 1 | 1 | 1 | 1 | 5 | 2 | 1 | 1 | 1 | 1 | 2 | 1 | 2 | 1 | 1 | 1 | 1 | 1 | 5 | 5 |
| | 3 | 1 | 1 | 1 | 1 | 1 | 1 | | 2 | 1 | 1 | 1 | 1 | 3 | 1 | 3 | 2 | 1 | 1 | 1 | 1 | | |
| | 2 | 1 | 1 | 1 | 1 | 1 | 1 | | 2 | 1 | 1 | 1 | 1 | 4 | 1 | 5 | 2 | 1 | 1 | 1 | 1 | | |

Tabelle 2 (Fortsetzung)

Selektivie herbizide Wirkung der erfindungsgemäßen Verbindungen in den Aufwandmengen 4, 3 und 2 kg a.i./ha vor dem Auflaufen (VA) bzw. nach dem Auflaufen (NA) der Testpflanzen

| Verbindung Nr. | kg Wirkstoff a.i./ha | SINAP | | LYCES | | ECHCE | | TRIAE | | AVEFA | | ALDMY | | GLYMA | | LOLPE | | STEME | | MATSS | | ORYSA | |
|---|---|---|---|---|---|---|---|---|---|---|---|---|---|---|---|---|---|---|---|---|---|---|---|
| | | VA | NA | VA | NA | VA | NA | VA | NA | VA | NA | VA | NA | VA | NA | VA | NA | VA | NA | VA | NA | VA | NA |
| 10 | 4<br>3<br>2 | 1<br>1<br>1 | 1<br>1<br>1 | 1<br>1<br>1 | 1<br>1<br>1 | 1<br>1<br>1 | 1<br>1<br>1 | 2<br>2<br>2 | 1<br>1<br>1 | 1<br>1<br>1 | 1<br>1<br>1 | 1<br>1<br>1 | 1<br>1<br>1 | 1<br>1<br>4 | 1<br>1<br>1 | 3<br>3<br>4 | 3<br>3<br>4 | 1<br>1<br>1 | 1<br>1<br>1 | 1<br>1<br>1 | 1<br>1<br>1 | 5 | 5 |
| 12 | 4<br>3<br>2 | 5 | 1<br>1<br>1 | 5 | 1<br>1<br>1 | 2<br>3<br>4 | 2<br>2<br>3 | 5 | 5 | 4<br>5 | 5 | 3<br>3<br>4 | 4<br>4<br>4 | 5 | 1<br>1<br>2 | 5 | 5 | 2<br>2<br>3 | 2<br>2<br>3 | 2<br>4<br>5 | 5 | 5 | 5 |
| 15 | 4<br>3<br>2 | 1<br>1<br>1 | 1<br>1<br>1 | 1<br>1<br>1 | 1<br>1<br>1 | 1<br>1<br>1 | 1<br>1<br>1 | 3<br>5 | 2<br>2<br>2 | 1<br>1<br>1 | 1<br>1<br>1 | 1<br>1<br>1 | 1<br>1<br>1 | 2<br>2<br>2 | 1<br>1<br>1 | 2<br>2<br>2 | 2<br>2<br>3 | 1<br>1<br>1 | 1<br>1<br>1 | 1<br>1<br>1 | 1<br>1<br>1 | 5 | 5 |
| 17 | 4<br>3<br>2 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>5 | 1<br>1<br>1 | 4<br>5 | 1<br>1<br>1 | 1<br>1<br>1 | 1<br>1<br>1 | 1<br>1<br>1 | 1<br>1<br>1 | 5 | 5 |
| 18 | 4<br>3<br>2 | 1<br>1<br>5 | 1<br>1<br>1 | 1<br>1<br>1 | 1<br>1<br>1 | 4<br>4<br>5 | 1<br>1<br>4 | 5 | 5 | 5 | 4 | 1<br>1<br>1 | 1<br>1<br>1 | 3<br>4<br>5 | 1<br>1<br>1 | 5 | 1<br>1<br>1 | 1<br>1<br>1 | 1<br>1<br>1 | 1<br>1<br>1 | 1<br>1<br>1 | 5 | 5 |
| 21 | 4<br>3<br>2 | 1<br>1<br>1 | 1<br>1<br>1 | 1<br>1<br>1 | 1<br>1<br>1 | 1<br>1<br>1 | 1<br>1<br>1 | 4<br>5 | 4<br>4<br>5 | 1<br>1<br>1 | 1<br>1<br>1 | 1<br>1<br>1 | 1<br>1<br>1 | 2<br>2<br>4 | 1<br>1<br>1 | 2<br>2<br>3 | 1<br>1<br>1 | 1<br>1<br>1 | 1<br>1<br>1 | 1<br>1<br>1 | 1<br>1<br>1 | 5 | 5 |
| 22 | 4<br>3<br>2 | 1<br>1<br>1 | 1<br>1<br>1 | 1<br>1<br>2 | 1<br>1<br>1 | 4<br>4<br>5 | 1<br>1<br>4 | | | 5 | 1<br>1<br>1 | 1<br>2<br>5 | 1<br>1<br>2 | 5 | 1<br>1<br>1 | 3<br>3<br>5 | 2<br>2<br>2 | 1<br>1<br>1 | 1<br>1<br>1 | 1<br>1<br>4 | 1<br>1<br>1 | 5 | 5 |
| 46 | 4<br>3<br>2 | 2<br>2<br>4 | 1<br>1<br>1 | 1<br>1<br>4 | 1<br>1<br>1 | 3<br>3<br>3 | 2<br>3<br>5 | 1<br>1<br>1 | 1<br>1<br>4 | 1<br>1<br>1 | 1<br>1<br>1 | 1<br>1<br>3 | 1<br>1<br>1 | 4<br>5 | 3<br>3<br>4 | 5 | 5 | 1<br>1<br>1 | 1<br>1<br>1 | 1<br>1<br>1 | 1<br>1<br>1 | 5 | 5 |
| 73 | 4<br>3<br>2 | 1<br>1<br>1 | 1<br>1<br>1 | 1<br>1<br>1 | 1<br>1<br>1 | 3<br>5 | 3<br>3<br>5 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 5 | 5 |

Tabelle 2 (Fortsetzung)

Selektivie herbizide Wirkung der erfindungsgemäßen Verbindungen in den Aufwandmengen 4, 3 und 2 kg a.i./ha vor dem Auflaufen (VA) bzw. nach dem Auflaufen (NA) der Testpflanzen

| Verbindung Nr. | kg Wirkstoff a.i./ha | SINAP | | LYCES | | ECHCE | | TRIAE | | AVEFA | | ALOMY | | GLYMA | | LOLPE | | STEME | | MATSS | | ORYSA | |
|---|---|---|---|---|---|---|---|---|---|---|---|---|---|---|---|---|---|---|---|---|---|---|---|
| | | VA | NA | VA | NA | VA | NA | VA | NA | VA | NA | VA | NA | VA | NA | VA | NA | VA | NA | VA | NA | VA | NA |
| 74 | 4<br>3<br>2 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 2<br>2<br>2 | 1<br>1<br>1 | 3<br>3<br>3 | 1<br>1<br>1 | 1<br>1<br>1 | 1<br>1<br>1 | 1<br>1<br>1 | 1<br>1<br>1 | 1<br>1<br>5 | 1<br>1<br>1 |
| 75 | 4<br>3<br>2 | 2<br>2<br>4 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 1<br>1<br>1 | 2<br>3<br>5 | 1<br>1<br>5 | 4<br>4<br>5 | 2<br>2<br>5 | 1<br>1<br>1 | 1<br>1<br>1 | 1<br>1<br>1 | 1<br>1<br>1 | 5 | 3<br>5 |
| 76 | 4<br>3<br>2 | 1<br>[illegible]<br>4 | 1<br>1<br>1 | 1<br>1<br>1 | 1<br>1<br>1 | 1<br>1<br>1 | 2<br>2<br>2 | 5 | 2<br>3<br>5 | 1<br>1<br>4 | 1<br>3<br>3 | 1<br>1<br>1 | 1<br>1<br>1 | 4<br>5 | 3<br>3<br>3 | 4<br>5 | 3<br>5 | 1<br>1<br>1 | 1<br>1<br>1 | 1<br>1<br>1 | 1<br>1<br>1 | 5 | 5 |
| 78 | 4<br>3<br>2 | 1<br>2<br>2 | 1<br>1<br>1 | 1<br>1<br>1 | 1<br>1<br>1 | 1<br>1<br>1 | 1<br>1<br>1 | 4<br>5 | 3<br>5 | 1<br>1<br>2 | 2<br>2<br>4 | 1<br>1<br>2 | 1<br>1<br>2 | 4<br>4<br>5 | 3<br>3<br>3 | 5 | 5 | 1<br>1<br>1 | 1<br>1<br>1 | 1<br>1<br>1 | 1<br>1<br>1 | 5 | 5 |
| 79 | 4<br>3<br>2 | 1<br>1<br>1 | 1<br>1<br>1 | 1<br>1<br>1 | 1<br>1<br>1 | 1<br>1<br>1 | 1<br>1<br>1 | 3<br>5 | 3<br>3<br>5 | 1<br>1<br>1 | 1<br>1<br>1 | 1<br>1<br>2 | 1<br>1<br>1 | 4<br>4<br>5 | 3<br>3<br>3 | 1<br>3<br>5 | 3<br>5 | 1<br>1<br>1 | 1<br>1<br>1 | 1<br>1<br>1 | 1<br>1<br>1 | 5 | 5 |
| 81 | 4<br>3<br>2 | 2<br>5 | 1<br>1<br>1 | 1<br>1<br>1 | 1<br>1<br>1 | 1<br>1<br>1 | 1<br>1<br>1 | 3<br>5 | 2<br>2<br>5 | 1<br>1<br>1 | 1<br>1<br>1 | 1<br>1<br>1 | 1<br>1<br>1 | 3<br>4<br>4 | 2<br>2<br>2 | 1<br>4<br>5 | 2<br>2<br>5 | 1<br>1<br>1 | 1<br>1<br>1 | 1<br>1<br>1 | 1<br>1<br>1 | 5 | 4<br>5 |
| 86 | 4<br>3<br>2 | 2<br>2<br>5 | 1<br>1<br>1 | 1<br>1<br>2 | 1<br>1<br>1 | 4<br>4<br>4 | 1<br>2<br>3 | 1<br>2<br>5 | 1<br>1<br>1 | 1<br>1<br>1 | 1<br>1<br>1 | 1<br>1<br>1 | 1<br>1<br>1 | 2<br>3<br>3 | 1<br>1<br>3 | 5 | 5 | 1<br>1<br>1 | 1<br>1<br>1 | 1<br>1<br>1 | 1<br>1<br>1 | 5 | 5 |
| 89 | 4<br>3<br>2 | 2<br>2<br>2 | 1<br>1<br>1 | 1<br>1<br>1 | 1<br>1<br>1 | 4<br>4<br>4 | 1<br>2<br>3 | 1<br>2<br>5 | 1<br>1<br>1 | 2<br>3<br>3 | 1<br>1<br>1 | 1<br>1<br>1 | 1<br>1<br>1 | 4<br>4<br>4 | 1<br>1<br>1 | 5 | 5 | 1<br>1<br>1 | 1<br>1<br>1 | 1<br>1<br>1 | 1<br>1<br>1 | 5 | 5 |

Tabelle 3

Selektive herbizide Wirkung der erfindungsgemäßen Verbindungen in den Aufwandmengen 1, 0,5, 0,25 und 0,125 kg a.i./ha vor dem Auflaufen (VA) bzw. nach dem Auflaufen (NA) der Testpflanzen

| Verbindung Nr. | kg Wirkstoff a.i./ha | ALOMY | | AMARE | | APESV | | AVEFA | | AVESA | | BETVU | | BRANA | | CHEAL | | DIGSA | | ECHCG | | GLYMA | |
|---|---|---|---|---|---|---|---|---|---|---|---|---|---|---|---|---|---|---|---|---|---|---|---|
| | | VA | NA | VA | NA | VA | NA | VA | NA | VA | NA | VA | NA | VA | NA | VA | NA | VA | NA | VA | NA | VA | NA |
| A (bekannt) | 1 | 1 | 1 | 2 | 1 | 1 | 1 | 1 | 1 | 1 | 1 | 1 | 1 | 1 | 1 | 1 | 1 | 1 | 1 | 1 | 1 | 5 | 1 |
| | 0,5 | 1 | 1 | 5 | 1 | 1 | 1 | 1 | 1 | 1 | 1 | 4 | 1 | 2 | 1 | 1 | 1 | 2 | 1 | 1 | 1 | | 1 |
| | 0,25 | 1 | 1 | | 1 | 2 | 1 | 2 | 1 | 1 | 1 | 5 | 1 | 4 | 1 | 2 | 1 | 3 | 1 | 2 | 1 | | 1 |
| | 0,125 | 2 | 3 | | 2 | 2 | 3 | 3 | 4 | 3 | 2 | | 1 | 5 | 5 | 3 | 2 | 3 | 2 | 4 | 1 | | 3 |
| B (bekannt) | 1 | 1 | 1 | 4 | 1 | 1 | 1 | 1 | 1 | 1 | 1 | 1 | 1 | 3 | 1 | 1 | 1 | 1 | 1 | 1 | 1 | 4 | 1 |
| | 0,5 | 1 | 1 | 4 | 5 | 1 | 1 | 3 | 1 | 1 | 1 | 1 | 1 | 3 | 4 | 1 | 1 | 2 | 1 | 2 | 1 | 5 | 1 |
| | 0,25 | 4 | 2 | 5 | | 3 | 2 | 3 | 3 | 3 | 2 | 4 | 1 | 3 | 4 | 2 | 1 | 4 | 3 | 4 | 1 | | 3 |
| | 0,125 | 5 | 3 | | | 5 | 2 | 4 | 5 | 5 | 4 | 5 | 2 | 4 | 5 | 4 | 3 | 4 | 3 | 5 | 3 | | 4 |
| C (bekannt) Atrazin | 1 | 1 | 1 | 1 | 1 | 1 | 1 | 1 | 1 | 1 | 1 | 1 | 1 | 1 | 1 | 1 | 1 | 1 | 3 | 1 | 1 | 1 | 1 |
| | 0,5 | 1 | 1 | 1 | 1 | 1 | 1 | 1 | 1 | 1 | 1 | 1 | 1 | 1 | 1 | 1 | 1 | 2 | 5 | 1 | 1 | 1 | 1 |
| | 0,25 | 2 | 1 | 1 | 1 | 1 | 1 | 1 | 1 | 1 | 1 | 1 | 1 | 1 | 1 | 1 | 1 | 4 | | 2 | 1 | 1 | 3 |
| | 0,125 | 2 | 1 | 1 | 1 | 1 | 1 | 4 | 1 | 1 | 1 | 1 | 1 | 2 | 1 | 1 | 1 | 4 | | 2 | 2 | 5 | 3 |
| 1 | 1 | 1 | 1 | 1 | 1 | 1 | 1 | 1 | 1 | 1 | 1 | 1 | 1 | 2 | 1 | 1 | 1 | 1 | 1 | 1 | 1 | 3 | 3 |
| | 0,5 | 1 | 1 | 3 | 1 | 1 | 1 | 1 | 1 | 1 | 1 | 1 | 1 | 2 | 2 | 1 | 1 | 1 | 1 | 1 | 1 | 3 | 4 |
| | 0,25 | 1 | 1 | 3 | 4 | 1 | 1 | 3 | 1 | 3 | 1 | 1 | 4 | 2 | 4 | 1 | 1 | 1 | 1 | 2 | 1 | 4 | 5 |
| | 0,125 | 2 | 2 | 4 | 4 | 2 | 2 | 3 | 5 | 3 | 2 | 3 | 4 | 3 | 5 | 2 | 1 | 4 | 2 | 4 | 1 | | |
| 2 | 1 | 1 | 1 | 1 | 1 | 1 | 1 | 1 | 1 | 1 | 1 | 1 | 1 | 1 | 1 | 1 | 1 | 1 | 1 | 1 | 1 | 5 | 1 |
| | 0,5 | 1 | 1 | 1 | 1 | 1 | 1 | 1 | 1 | 1 | 1 | 1 | 1 | 1 | 1 | 1 | 1 | 1 | 1 | 1 | 1 | | 1 |
| | 0,25 | 1 | 1 | 1 | 1 | 1 | 1 | 2 | 1 | 1 | 1 | 2 | 1 | 2 | 1 | 1 | 1 | 1 | 1 | 2 | 1 | | 1 |
| | 0,125 | 1 | 1 | 5 | 2 | 1 | 1 | 2 | 3 | 3 | 2 | 4 | 1 | 3 | 1 | 1 | 1 | 2 | 1 | 2 | 1 | | 4 |
| 3 | 1 | 1 | 1 | 1 | 1 | 1 | 1 | 1 | 1 | 1 | 1 | 1 | 1 | 1 | 1 | 1 | 1 | 1 | 1 | 1 | 1 | 3 | 1 |
| | 0,5 | 1 | 1 | 3 | 1 | 1 | 1 | 1 | 1 | 1 | 1 | 1 | 1 | 2 | 1 | 1 | 1 | 1 | 1 | 1 | 1 | 5 | 1 |
| | 0,25 | 1 | 1 | 5 | 1 | 1 | 1 | 1 | 1 | 1 | 1 | 1 | 1 | 4 | 1 | 1 | 1 | 1 | 1 | 1 | 1 | | 1 |
| | 0,125 | 2 | 1 | | 1 | 1 | 1 | 1 | 1 | 1 | 1 | 1 | 1 | 5 | 1 | 1 | 1 | 3 | 1 | 4 | 1 | | 3 |
| 74 | 1 | 1 | 1 | 1 | 1 | 1 | 1 | 1 | 1 | 1 | 1 | 1 | 1 | 1 | 1 | 1 | 1 | 1 | 1 | 1 | 1 | 2 | 1 |
| | 0,5 | 1 | 1 | 3 | 1 | 1 | 1 | 1 | 1 | 1 | 1 | 1 | 1 | 1 | 1 | 1 | 1 | 1 | 1 | 1 | 1 | 2 | 2 |
| | 0,25 | 1 | 1 | 5 | 1 | 1 | 1 | 1 | 1 | 3 | 2 | 1 | 1 | 2 | 1 | 1 | 1 | 1 | 1 | 1 | 1 | 5 | 5 |
| | 0,125 | 4 | 1 | | 1 | 1 | 1 | 1 | 1 | 3 | 2 | 4 | 1 | 4 | 1 | 1 | 1 | 2 | 1 | 1 | 1 | | |

0126780

**Tabelle 3** (Fortsetzung)

Selektive herbizide Wirkung der erfindungsgemäßen Verbindungen in den Aufwandmengen 1, 0,5, 0,25 und 0,125 kg a.i./ha vor dem Auflaufen (VA) bzw. nach dem Auflaufen (NA) der Testpflanzen

| Verbindung Nr. | kg Wirkstoff a.i./ha | GOSHI | | HORVU | | LAMPU | | LOLPE | | LYCES | | MATSS | | ORYSA | | SECSE | | SETFA | | SETVI | | SINAP | |
|---|---|---|---|---|---|---|---|---|---|---|---|---|---|---|---|---|---|---|---|---|---|---|---|
| | | VA | NA | VA | NA | VA | NA | VA | NA | VA | NA | VA | NA | VA | NA | VA | NA | VA | NA | VA | NA | VA | NA |
| A (bekannt) | 1<br>0,5<br>0,25<br>0,125 | 5 | 4<br>5 | 4<br>5 | 2<br>3<br>5 | 1<br>1<br>1<br>2 | 1<br>1<br>1<br>2 | 5 | 2<br>4<br>5 | 1<br>1<br>2<br>4 | 1<br>1<br>1<br>1 | 1<br>1<br>1<br>1 | 1<br>1<br>1<br>1 | 3<br>4<br>5 | 5 | 3<br>5 | 2<br>4<br>5 | 1<br>1<br>3<br>3 | 1<br>1<br>2<br>3 | 1<br>1<br>2<br>3 | 1<br>1<br>2<br>3 | 1<br>1<br>3<br>3 | 1<br>1<br>1<br>1 |
| B (bekannt) | 1<br>0,5<br>0,25<br>0,125 | 5 | 5 | 5 | 1<br>2<br>5 | 1<br>1<br>1<br>3 | 1<br>1<br>2<br>3 | 5 | 5 | 1<br>1<br>2<br>4 | 1<br>1<br>1<br>2 | 1<br>1<br>1<br>1 | 1<br>1<br>1<br>1 | 5 | 5 | 4<br>5 | 3<br>4<br>5 | 1<br>1<br>3<br>4 | 1<br>1<br>2<br>3 | 1<br>1<br>3<br>3 | 1<br>1<br>1<br>3 | 2<br>2<br>5 | 1<br>1<br>4<br>5 |
| C (bekannt) Atrazin | 1<br>0,5<br>0,25<br>0,125 | 2<br>4<br>5 | 2<br>3<br>5 | 1<br>1<br>3<br>3 | 1<br>1<br>3<br>3 | 1<br>1<br>1<br>1 | 1<br>1<br>1<br>1 | 1<br>2<br>3<br>3 | 1<br>1<br>4<br>4 | 1<br>1<br>1<br>1 | 1<br>1<br>1<br>1 | 1<br>1<br>1<br>1 | 1<br>1<br>1<br>1 | 1<br>1<br>3<br>4 | 1<br>1<br>2<br>4 | 2<br>3<br>3<br>3 | 1<br>3<br>4<br>4 | 3<br>3<br>4<br>4 | 1<br>1<br>2<br>4 | 1<br>1<br>3<br>3 | 1<br>1<br>3<br>3 | 1<br>1<br>1<br>1 | 1<br>1<br>1<br>1 |
| 1 | 1<br>0,5<br>0,25<br>0,125 | 5 | 5 | 4<br>5 | 2<br>3<br>5 | 1<br>1<br>1<br>1 | 1<br>1<br>1<br>1 | 2<br>4<br>5 | 3<br>3<br>4<br>5 | 1<br>1<br>4<br>5 | 1<br>1<br>4<br>4 | 1<br>1<br>1<br>1 | 1<br>1<br>1<br>1 | 5 | 5 | 3<br>5 | 4<br>5 | 1<br>1<br>1<br>3 | 1<br>1<br>2<br>2 | 1<br>1<br>2<br>2 | 1<br>1<br>1<br>1 | 1<br>1<br>3<br>3 | 1<br>1<br>1<br>1 |
| 2 | 1<br>0,5<br>0,25<br>0,125 | 5 | 4<br>5 | 5 | 2<br>3<br>5 | 1<br>1<br>1<br>1 | 1<br>1<br>1<br>1 | 5 | 1<br>2<br>4<br>5 | 1<br>1<br>1<br>1 | 1<br>1<br>1<br>1 | 1<br>1<br>1<br>1 | 1<br>1<br>1<br>1 | 3<br>4<br>5 | 4<br>5 | 5 | 3<br>5 | 1<br>1<br>2<br>2 | 1<br>1<br>1<br>2 | 1<br>1<br>1<br>2 | 1<br>1<br>1<br>1 | 1<br>1<br>2<br>2 | 1<br>1<br>1<br>1 |
| 3 | 1<br>0,5<br>0,25<br>0,125 | 4<br>5 | 5 | 5 | 1<br>2<br>5 | 1<br>1<br>1<br>1 | 1<br>1<br>1<br>1 | 5 | 1<br>2<br>4<br>5 | 1<br>1<br>1<br>4 | 1<br>1<br>1<br>1 | 1<br>1<br>1<br>1 | 1<br>1<br>1<br>1 | 5 | 5 | 5 | 3<br>4<br>5 | 1<br>1<br>1<br>2 | 1<br>1<br>1<br>1 | 1<br>1<br>1<br>2 | 1<br>1<br>1<br>2 | 1<br>1<br>1<br>2 | 1<br>1<br>1<br>1 |
| 74 | 1<br>0,5<br>0,25<br>0,125 | 5 | 5 | 4<br>5 | 1<br>2<br>5 | 1<br>1<br>1<br>1 | 1<br>1<br>1<br>1 | 3<br>4<br>4<br>5 | 4<br>5 | 1<br>1<br>1<br>1 | 1<br>1<br>1<br>1 | 1<br>1<br>1<br>1 | 1<br>1<br>1<br>1 | 5 | 3<br>5 | 3<br>5 | 2<br>3<br>5 | 1<br>1<br>1<br>3 | 1<br>1<br>1<br>1 | 1<br>1<br>1<br>2 | 1<br>1<br>1<br>1 | 1<br>1<br>1<br>2 | 1<br>1<br>1<br>1 |

Tabelle 3 (Fortsetzung)

Selektive herbizide Wirkung der erfindungsgemäßen Verbindungen in den Aufwandmengen 1, 0,5, 0,25 und 0,125 kg a.i./ha vor dem Auflaufen (VA) bzw. nach dem Auflaufen (NA) der Testpflanzen

| Verbindung Nr. | kg Wirkstoff a.i./ha | STEME | | TRIAE | | ZEAMA | | | | | | | | | | | | | | | | | |
|---|---|---|---|---|---|---|---|---|---|---|---|---|---|---|---|---|---|---|---|---|---|---|---|
| | | VA | NA | VA | NA | VA | NA | VA | NA | VA | NA | VA | NA | VA | NA | VA | NA | VA | NA | VA | NA | VA | NA |
| A (bekannt) | 1 | 1 | 1 | 4 | 1 | 5 | 4 | | | | | | | | | | | | | | | | |
| | 0,5 | 1 | 1 | 5 | 1 | | 5 | | | | | | | | | | | | | | | | |
| | 0,25 | 1 | 1 | | 3 | | | | | | | | | | | | | | | | | | |
| | 0,125 | 2 | 1 | | 4 | | | | | | | | | | | | | | | | | | |
| B (bekannt) | 1 | 1 | 1 | 4 | 2 | 4 | 3 | | | | | | | | | | | | | | | | |
| | 0,5 | 1 | 1 | 5 | 3 | 5 | 4 | | | | | | | | | | | | | | | | |
| | 0,25 | 1 | 1 | | 5 | | 4 | | | | | | | | | | | | | | | | |
| | 0,125 | 1 | 1 | | | | 5 | | | | | | | | | | | | | | | | |
| C (bekannt) Atrazin | 1 | 1 | 1 | 2 | 2 | 5 | 5 | | | | | | | | | | | | | | | | |
| | 0,5 | 1 | 1 | 2 | 2 | | | | | | | | | | | | | | | | | | |
| | 0,25 | 1 | 1 | 2 | 2 | | | | | | | | | | | | | | | | | | |
| | 0,125 | 1 | 1 | 2 | 2 | | | | | | | | | | | | | | | | | | |
| 1 | 1 | 1 | 1 | 4 | 1 | 5 | 5 | | | | | | | | | | | | | | | | |
| | 0,5 | 1 | 1 | 5 | 1 | | | | | | | | | | | | | | | | | | |
| | 0,25 | 1 | 1 | | 3 | | | | | | | | | | | | | | | | | | |
| | 0,125 | 2 | 1 | | 5 | | | | | | | | | | | | | | | | | | |
| 2 | 1 | 1 | 1 | 5 | 2 | 3 | 4 | | | | | | | | | | | | | | | | |
| | 0,5 | 1 | 1 | | 3 | 3 | 4 | | | | | | | | | | | | | | | | |
| | 0,25 | 1 | 1 | | 5 | 4 | 4 | | | | | | | | | | | | | | | | |
| | 0,125 | 1 | 1 | | | 4 | 4 | | | | | | | | | | | | | | | | |
| 3 | 1 | 1 | 1 | 4 | 1 | 2 | 4 | | | | | | | | | | | | | | | | |
| | 0,5 | 1 | 1 | 5 | 1 | 5 | 5 | | | | | | | | | | | | | | | | |
| | 0,25 | 1 | 1 | | 3 | | | | | | | | | | | | | | | | | | |
| | 0,125 | 1 | 1 | | 5 | | | | | | | | | | | | | | | | | | |
| 74 | 1 | 1 | 1 | 4 | 3 | 2 | 4 | | | | | | | | | | | | | | | | |
| | 0,5 | 1 | 1 | 5 | 3 | 2 | 5 | | | | | | | | | | | | | | | | |
| | 0,25 | 1 | 1 | | 3 | 5 | | | | | | | | | | | | | | | | | |
| | 0,125 | 1 | 1 | | 3 | | | | | | | | | | | | | | | | | | |

Tabelle 3: Selektive herbizide Wirkung der erfindungsgemäßen Verbindungen in Paddy-Reis

| Verbindung Nr. | kg Wirkstoff a.i./ha | Wirkungsgrad (%) Reis | Echinochloa crus-galli |
|---|---|---|---|
| 1 | 3<br>2<br>1<br>0,5 | 38<br>28<br>0<br>0 | 100<br>100<br>92<br>92 |
| 2 | 3<br>2<br>1<br>0,5 | 41<br>18<br>5<br>0 | 100<br>100<br>100<br>100 |
| 3 | 3<br>2<br>1<br>0,5 | 38<br>14<br>0<br>0 | 100<br>100<br>100<br>100 |
| 4 | 3<br>2<br>1<br>0,5 | 23<br>0<br>0<br>0 | 100<br>100<br>100<br>100 |
| 73 | 3<br>2<br>1<br>0,5 | 0<br>0<br>0<br>0 | 100<br>100<br>99<br>76 |
| 74 | 3<br>2<br>1<br>0,5 | 0<br>0<br>0<br>0 | 100<br>100<br>88<br>92 |
| 75 | 3<br>2<br>1<br>0,5 | 0<br>0<br>0<br>0 | 98<br>94<br>64<br>41 |
| A (bekannt) | 3<br>2<br>1<br>0,5 | 41<br>21<br>0<br>0 | 100<br>100<br>90<br>83 |
| B (bekannt) | 3<br>2<br>1<br>0,5 | 0<br>0<br>0<br>0 | 99<br>89<br>42<br>5 |
| C (bekannt) | 3<br>2<br>1<br>0,5 | 100<br>100<br>100<br>100 | 100<br>100<br>100<br>100 |
| D (bekannt) | 3<br>2<br>1<br>0,5 | 100<br>100<br>100<br>100 | 100<br>100<br>100<br>100 |
| E (bekannt) | 3<br>2<br>1<br>0,5 | 0<br>0<br>0<br>0 | 100<br>100<br>96<br>85 |

Patentansprüche:

1. Verwendung von Alkoxy-cycloalkylamino-s-triazinen der allgemeinen Formel

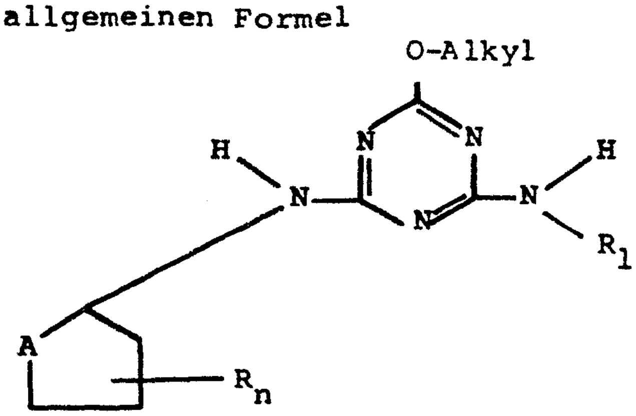

in der

$R_1$ einen gerad- oder verzweigtkettigen Alkyl-, Alkoxy-, Alkoxyalkyl- oder Alkylol-Rest oder ein H-Atom,

R einen Rest aus der Gruppe der Alkyle mit ein bis drei C-Atomen und

A = $-CH_2-$ oder $-CH_2-CH_2-$ bedeuten und

n für A = $-CH_2-$ jeweils 0 oder 1,2,3,4,5 oder 6, bzw. für A = $-CH_2-CH_2-$ jeweils 3,4,5 oder 6

sein können und wobei die Substituenten R an den Cycloalkylringen gleich oder verschieden sein können, in geeigneter Applikationsform zur selektiven Behandlung von Nutzpflanzenkulturen mit breitem Wirkungsspektrum gegen Unkräuter und Schadgräser

vor und/oder nach dem Auflaufen der Saat.

2. Verwendung von Mischungen von zwei oder mehreren Wirkstoffen nach Anspruch 1 gemäß Formel I, bei denen sich die Einzelwirkstoffe durch unterschiedliche Substituenten $R_1$ und/oder R und/oder von A und/oder die Werte von n voneinander unterscheiden.

3. Verwendung eines oder mehrerer der Wirkstoffe nach Anspruch 1 oder 2 in Mengen von je 0,05 bis 10 kg/ha, vorzugsweise von 0,5 bis 2,5 kg/ha.

4. Verwendung eines oder mehrerer der Wirkstoffe nach Anspruch 1 oder 2 und 3,
dadurch gekennzeichnet,
daß man die jeweilige Applikationsform durch Zumischen von Hilfsmitteln aus der Gruppe der Träger-, Verdünnungs-, Lösungs-, Netz-, Haft-, Dispergier- und Emulgiermittel gewinnt.

5. Verwendung eines oder mehrerer der Wirkstoffe nach Anspruch 1 und einem oder mehreren der Ansprüche 2 bis 4,
gekennzeichnet durch
die Zumischung von Stoffen aus der Gruppe der Herbizide, Fungizide, Insektizide, Wachstumsregulatoren usw.

6. Verwendung eines oder mehrerer der Wirkstoffe nach Anspruch 1 und gegebenenfalls nach einem oder mehreren der Ansprüche 2 bis 5
gekennzeichnet durch
den Zusatz von Mineraldüngern.

Europäisches Patentamt

# EUROPÄISCHER RECHERCHENBERICHT

0167730
Nummer der Anmeldung

EP 85 10 4950

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| Y | EP-A-0 096 189 (CHEMISCHE WERKE HÜLS)<br>* Patentansprüche * & DE - A - 3 222 147 (Kat. D)<br>--- | 1-6 | A 01 N 43/70<br>C 07 D 251/52 |
| Y | CH-A- 337 019 (J.R. GEIGY)<br>* Patentansprüche; Seite 2, Zeile 8 *<br>--- | 1-6 | |
| Y | CENTRAL PATENTS INDEX, BASIC ABSTRACTS JOURNAL, Section C: Agdoc, Woche T27, 1972, Nr. 44009T, Derwent Publications, London, GB; & JP - A - 72 23 436 (NIPPON KAYAKU) 30.06.1972<br>--- | 1-6 | |
| A | EP-A-0 084 767 (CHEMISCHE WERKE HÜLS)<br>& DE - A - 3 202 085 (Kat. D)<br>----- | | RECHERCHIERTE SACHGEBIETE (Int Cl 4)<br>A 01 N<br>C 07 D |

Der vorliegende Recherchenbericht wurde für alle Patentansprüche erstellt.

| Recherchenort | Abschlußdatum der Recherche | Prüfer |
|---|---|---|
| DEN HAAG | 17-09-1985 | DECORTE D. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503 03 82